(19) Europäisches Patentamt
European Patent Office
Office européen des brevets

(11) Publication number: 0 354 568
A2

# (12) EUROPEAN PATENT APPLICATION

(21) Application number: 89114800.9

(22) Date of filing: 10.08.89

(51) Int. Cl.4: C07D 211/58 , A61K 31/445

Claims for the following Contracting State: ES.

(30) Priority: 12.08.88 JP 200006/88

(43) Date of publication of application:
14.02.90 Bulletin 90/07

(84) Designated Contracting States:
AT BE CH DE ES FR GB IT LI NL SE

(71) Applicant: Japan Tobacco Inc.
2-1 Toranomon, 2-Chome
Minato-Ku Tokyo 105(JP)

Applicant: YOSHITOMI PHARMACEUTICAL INDUSTRIES, LTD.
6-9, Hiranomachi 2-chome Chuo-ku
Osaka-shi Osaka 541(JP)

(72) Inventor: Uchida, Itsuo c/o Pharmaceutical Research Lab.
Japan Tobacco Inc. 6-2, Umegaoka
Midori-ku Yokohama-shi Kanagawa, 227(JP)
Inventor: Shinagawa, Yukou c/o
Pharmaceutical Research Lab.
Japan Tobacco Inc. 6-2, Umegaoka
Midori-ku Yokohama-shi Kanagawa, 227(JP)
Inventor: Kishimoto, Toshimitsu Kiyose Dormitory Yoshitomi
Pharmaceutical Ind. Ltd. 12-19, Kamikiyoto 2-chome
Kiyose-shi Tokyo, 204(JP)
Inventor: Ono, Takashi Kiyose Dormitory Yoshitomi
Pharmaceutical Ind. Ltd. 12-19, Kamikiyoto 2-chome
Kiyose-shi Tokyo, 204(JP)
Inventor: Arita, Masafumi
Tokorozawa Maison No. 202 6-11, Minamisumiyoshi
Tokorozawa-shi Saitama, 359(JP)

(74) Representative: von Kreisler, Alek, Dipl.-Chem. et al
Patentanwälte Von Kreisler-Selting-Werner
Deichmannhaus am Hauptbahnhof
D-5000 Köln 1(DE)

## (54) Novel catechol derivatives.

(57) Catechol derivatives represented by the general formula:

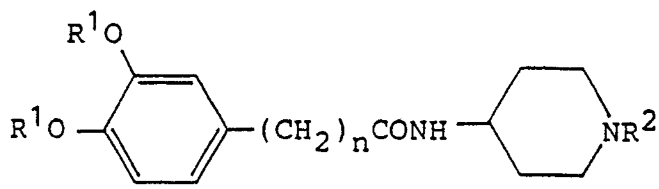

wherein each symbol is as defined in the specification and their pharmaceutically acceptable acid addition salts.
Said derivatives are useful for the prevention or treatment of degenerative neurological diseases.

EP 0 354 568 A2

# NOVEL CATECHOL DERIVATIVES

## BACKGROUND OF THE INVENTION

### 1. Field of the Invention:

This invention relates to novel catechol derivatives, particularly useful as a medicament for preventing or treating degenerative neurological disorders such as Alzheimer's disease, general senile dementia, etc. by promoting the production or excretion of nerve growth factor (hereinafter abbreviated as "NGF").

### 2. Statement of Related Art:

Of recent years, the presence and synthesis of NGF in the brain have been confirmed and its distribution has been proved to be localized in the cholinergic nervous system (S. Korsching et al, EMBO J., 4, 1389 - 1393 (1985)). This region is an important part for memory and learning, and NGF is known to be significantly degenerated or lost in Alzheimer type dementia. In vitro experiments, NGF has been reported to exhibit a life maintaining action or a choline acetyl-transferase activity-promoting action to central cholinergic neurons (H. Guahn et al, Dev. Brain Res., 9, 45 - 52 (1983)). Another paper has presented that also in animal experiments such as in Alzheimer's disease model rats whose each septal hippocampus projection system is disordered, senile rats having memory disorder, etc., the administration of NGF to the lateral ventricle permits to improve on the memory or learning ability (B. Will, Behav. Brain Res., 17, 17 - 24 (1985) and W. Fischer et al, Nature, 329, 65 - 68 (1987)).

Further, it has been reported that administration of NGF can prevent ischemic neurological disorders in the hippocampus (Kusano et al, IGAKU NO AYUMI (Progress in Medicine), 145, 579 - 580 (1988)).

From the foregoing findings, NGF or promotors for the production of NGF are expected to be an efficacious medicament for various central neurological disorders such as Alzheimer's disease.

As a compound promoting the production of NGF, catechol-amines and alkyl-substituted catechol derivatives have been reported (Y. Furukawa et al, J. Biol. Chem., 261, 6039 - 6047 (1986); Japanese Patent First Publication 63-83020 (1988); and Japanese Patent First Publication 63-156751 (1988)). Moreover, cinnamide derivatives having 5-riboxygenase-inhibiting action, which are analogous compounds to the catechol derivatives of this invention hereinbelow described, have already been disclosed (Japanese Patent First Publication 62-12757 (1987)).

The activity of the known catechol derivatives which have been found to promote the production of NGF in the foregoing references, however, is virtually insufficient and under the circumstances, useful compounds that have a higher activity are earnestly desired.

## SUMMARY OF THE INVENTION

With a view toward developing a preventive against the progress of central nervous system diseases or neurological disorders and a curing agent for them, whose action mechanism consists in promoting production of NGF, the present inventors have intensively investigated into catechol-series low molecular compounds thereby to find more useful compounds. As a result, certain new kinds of catechol amide compounds have been discovered to show a strong promoting action for the production of NGF, based on which this invention has been accomplished.

That is, this invention is designed to provide catechol derivatives represented by the general formula (I) given below and their pharmaceutically acceptable acid addition salts:

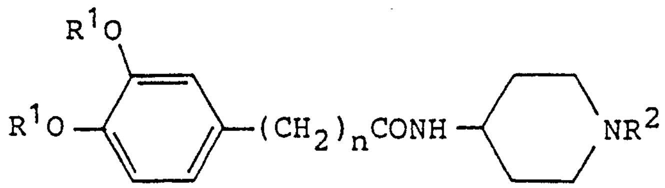

(I)

In formula (I), n is an integer of 1 to 4; $R^1$ is hydrogen atom, an acyl radical or a carbamoyl radical; $R^2$ is hydrogen atom, a lower alkyl radical or an aralkyl radical of the formula:

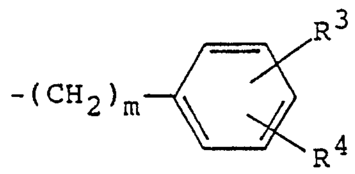

or

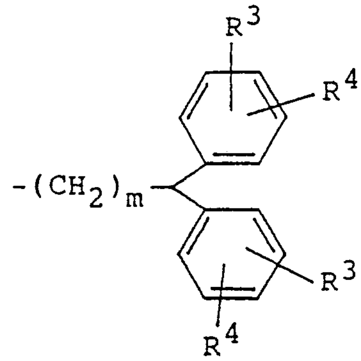

, wherein $R^3$ and R4$^4$may be the same or different substituents selected from the group consisting of hydrogen atom, a lower alkoxy, a halogen atom, a lower alkyl, trifluoromethyl and hydroxyl and m stands for an integer of 1 to 4.

In the definition above, the acyl radical includes an alkanoyl radical having 4 or less carbon atoms, e.g., acetyl, propionyl, etc. or an alkoxycarbonyl radical, e.g., methoxycarbonyl, ethoxycarbonyl, etc. The carbamoyl radical means a carbamoyl radical substituted by an alkyl having 6 or less carbon atoms, which includes butylaminocarbonyl, methylaminocarbonyl, etc. The halogen atom includes chlorine, fluorine, bromine or iodine. The lower alkyl radical is defined by an alkyl having 1 to 6 carbon atoms, e.g., methyl, ethyl, propyl, iso-propyl, butyl, iso-butyl, tert-butyl, pentyl, hexyl, etc. The lower alkoxy radical means an alkoxy having 1 to 6 carbon atoms and includes, for example, methoxy, ethoxy, propoxy, iso-propoxy, butoxy, iso-butoxy, tert-butoxy, pentyloxy, hexyloxy, etc.

The catechol derivatives of formula (I) according to this invention can be synthesized in four kinds of processes including A Process to D Process:

## A Process

This is based on hydrogenation of a compound of the formula (II):

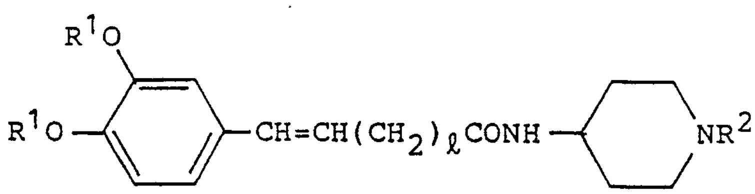

(II)

($\ell$ is an integer of 0 to 2, $R^1$ and $R^2$ are the same as above) or its acid addition salt by use of a catalyst (e.g., Pd/C, $Pd(OH)_2$, $PtO_2$).

Usually, a solvent to be used in this reduction process includes, for example, water, methanol, ethanol, iso-propyl alcohol, ethyl acetate, tetrahydrofuran, dioxane, dimethylformamide, acetic acid or a mixture thereof, but is not limited to them. The reduction is carried out under normal pressure or moderate pressure (3 to 5 atm.) at a suitable temperature extending from a cooling temperature to ca. 50$^\circ$ C. The reaction time is several ten minutes to several days. In case where $R^2$ of formula (II) is benzyl radical, this process affords

a compound of formula (I) wherein $R^2$ is hydrogen atom.

B Process

This process comprises making to react a carboxylic acid or its reactive derivative of the formula (III):

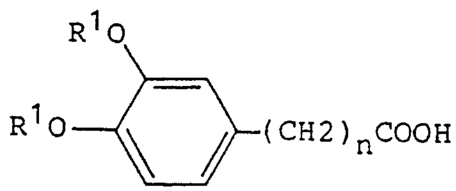

(III)

(R' and n are the same as above) and an amine of the formula (IV):

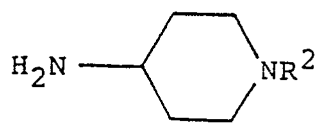

(IV)

($R^2$ is the same as above except for hydrogen atom).

In this process, all procedures for producing amide linkage can be applied. For instance, a carboxylic acid and an amine are made to react, usually in the presence of a dehydrating agent such as dicyclohexylcarbodiimide, N,N'-carbonyldiimidazole, etc. After a reactive derivative of a carboxylic acid is preliminarily produced or produced in the reaction system, it is possible to perform the reaction for producing amide linkage. Here, the reactive derivative of a carboxylic acid includes acid halides, acid anhydrates, mixed acid anhydrates obtained by the reaction with ethyl chlorocarboxylate and active ester compounds (p-nitrophenyl ester, N-hydroxysuccinimide ester, 1-hydroxybenzotriazole ester, N-hydroxy-5-norbornen-2,3-dicarboxylic imide ester).

Where a compound of formula (III) has a phenolic hydro xyl group, the hydroxyl group is protected appropriately by acylation with a lower aliphatic acid, by 2-pyranyloxylation with 2,3-dihydroxypyran, or by silylation with trimethylchlorosilane, t-butyldimethylchlorosilane, etc., and then, the reaction of amide formation can be effected. Therefore, the protective group can be removed by treatment with an alkali, acid and water, thereby to give the objective compounds of formula (I) (except for hydrogen atom for $R^2$).

The solvents which usually can be used in the respective reactions above include, for example, ethanol, iso-propanol, ethyl acetate, benzene, toluene, acetone, tetrahydrofuran, methylene chloride, dioxane, acetonitrile, dimethylformamide, dimethylsulfoxide or a mixture of them, but are not limited to them.

C Process

This process comprises reacting a compound of the formula (V):

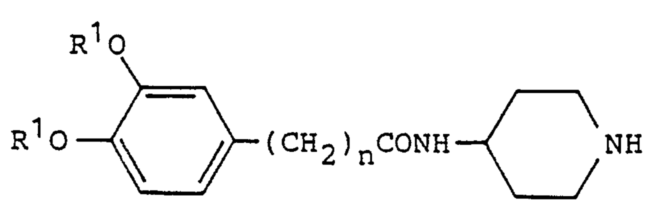

(V)

($R^1$ and n are the same as above) and a compound of the formula (VI):

$R^2$ - X (VI)

($R^2$ is the same as above except for hydrogen atom; X is a halogen atom such as bromine, iodine, chlorine or an active ester (mesyloxy, tosyloxy)).

This reaction can be carried out at a temperature of 0 -100 °C, in the presence of a base, e.g., potassium carbonate, sodium carbonate, potassium hydroxide, sodium hydroxide, sodium methylate, sodium ethylate, triethylamine, pyridine, 4,4-dimethylaminopyridine in a suitable solvent (methanol, ethanol, dimethylformamide, tetrahydrofuran, dimethoxyethane, ether, dimethylsulfoxide, etc.).

The compounds of formula (V) can be readily obtained by hydrogenating a compound of the formula (VII) given below, which is obtained in B Process, in a similar procedure to A Process in the presence of a catalyst, e.g., Pd/C, $Pd(OH)^2$ etc.

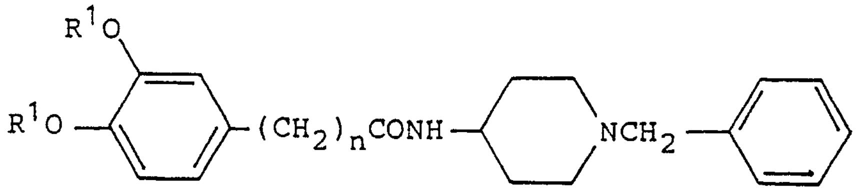

(VII)

D Process

In the production of the compounds of formula (I) wherein $R^2$ is an acyl or a carbamoyl, the following processes can be applied, starting from a compound of the formula (VIII):

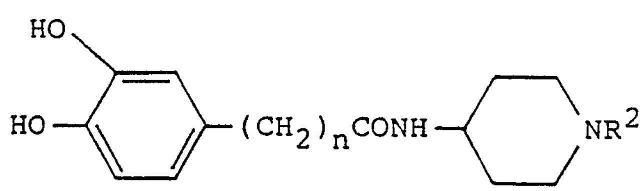

(VIII)

($R^2$ and n have the same definition above).

(1) Where R' is an acyl:

The objective compounds can be produced by the reaction of a compound of formula (VIII) with a corresponding lower aliphatic acid or its acid anhydride in the presence of an acid catalyst (mineral acids, e.g., sulphuric acid, hydrohalogenic acid; Lewis acid, e.g., anhydrous aluminum chloride, zinc chloride, ferric chloride, titanium tetrachloride, tin tetrachloride, $BF_3$, etc.) by direct heating reaction with a lower aliphatic acid anhydride, or by reaction with a lower aliphatic acid, or its anhydride or its chloride in the presence of a base (sodium hydroxide, potassium hydroxide, sodium carbonate, sodium bicarbonate, triethylamine, pyridine, etc.).

(2) Where R' is a carbamoyl:

The objective compounds can be produced by the reaction between a compound of formula (VIII) and a lower alkyl-substituted isocyanate at room temperature or under heating in the presence of a base catalyst such as triethylamine, pyridine, sodium acetate, etc. or in the presence of an acid catalyst such as borontrifluoride etherate, aluminum chloride, hydrochloric acid, trichloroacetic acid, etc, as the case may be.

The compounds of formula (I) thus obtained may be made their pharmaceutically acceptable salts by addition to inorganic acids, e.g., hydrochloric acid, hydrobromic acid, sulphuric acid, nitric acid, phosphoric acid or organic acids, e.g., p-toluene sulfonic acid, methane sulfonic acid, citric acid, lactic acid, maleic acid, fumaric acid, tartaric acid, etc.

The pharmaceutical composition for the prevention or treatment of the degenerative neurological disorders comprises a therapeutically effective amount of the compounds of the present invention mixed with pharmaceutically acceptable additives such as carrier, excipient or diluent. The pharmaceutical composition includes tablets, glanules, capsules, powder, solution, injectable solution, instillation agent,

suppository, etc. and can be administered to the patients in need of such therapy without harmful adverse effects. The daily dose may vary depending upon the symptoms, the kind of compounds to be administered, the body weights or ages of the patients, and preferably ranges about 1 to 50 mg/kg by the oral administration for human adults.

| Pharmaceutical Composition 1: | |
|---|---|
| Compound of formula (I) | 2.5 mg |
| Crystalline cellulose | 67.5 mg |
| Corn starch | 25.0 mg |
| Talc | 4.0 mg |
| Magnesium stearate | 1.0 mg |

The ingredients are mixed sufficiently, granulated, dried, and shaped into tablets weighing each 100 mg with the aid of a punch. The tablets can be subjected to sugar-coating or film-coating treatment in a conventional manner to produce sugar-coated tablets or film-coated tablets.

| Pharmaceutical Composition 2 | |
|---|---|
| Compound of formula (I) | 5.0 mg |
| Lactic acid | 22.0 mg |
| Crystalline cellulose | 7.5 mg |
| Carboxymethyl cellulose | 2.0 mg |
| Talc | 3.0 mg |
| Magnesium stearate | 0.5 mg |

The ingredients above are mixed and then encapsulated with a capsule filler.

According to the technique of Furukawa et al (J. Biol. Chem., 261, 6039 - 6047 (1986), Biochem. Biophys. Commun., 147, 1048 - 1054 (1987)), promoting action of NGF production of the compounds relating to this invention was examined by use of mouse fibroblast cell line, namely L-M cells (ATCC CCL 1•2).

The results obtained showed that the compounds of this invention exhibit a remarkable promoting action of NGF production.

From these experimental examples, the compounds of formula (I) and their pharmaceutically acceptable acid addition salts were proved to be novel compounds having a remarkable promoting action of NGF production. Serving to promote the production and excretion of NGF thereby to retain the living of neurons, regenerate the function of them or recontrol them by un-degenerated neurons, they are useful as a prophylactic, a progress-prohibitor and a curing agent for degenerative neurological diseases or disorders.

## PREFERRED EMBODIMENTS

This invention will be hereinafter described in more detail with reference to the following examples, but is not limited to them.

### Example 1

N-[1-(3-Phenylpropyl)-4-pyridyl,-3,4-dihydroxycinnamide•hydrochloride•monohydrate, 0.74 g, was dissolved in 30 ml of methanol, followed by degassing. To the solution was added 100 mg of 5% palladium charcoal and hydrogenation was performed at room temperature under normal pressure for 3 hours. After the reaction was completed, the palladium charcoal was filtered off and the filtrate was concentrated to

dryness under reduced pressure to yield 0.67 g of N-[1 (3-phenylpropyl)-4-piperidyl-3-(3,4 dihydroxyphenyl)propionamide•hydrochloride (colorless powder, m.p. 88 - 93 °C). NMR Spectra ($CD_3OD$)
δ: 1.60 - 1.82 (2H, m), 1.93 - 2.20 (4H, m), 2.40 (2H, t, J = 7.3Hz), 2.69 (2H, t, J = 8.2Hz), 2.75 (2H, t, J = 7.4Hz), 2.90 - 3.18 (4H, m), 3.77 - 3.92 (1H, m), 6.50 - 6.73 (3H, m), 7.14 - 7.40 (5H, m)
MASS Spectrum m/z 383 ($MH^+$)

Example 2

N-[1-(3-(4-methoxyphenyl)propyl)-4-piperidyl]-3,4-dihydroxycinnamide•hydrochloride, 200 mg, was dissolved in 6 ml of methanol. After outgassing, to the solution was added 20 mg of 5% palladium charcoal and the mixture was subjected to hydrogenation at room temperature under normal pressure for 2 hours. After the completion of the reaction, the palladium charcoal was filtered off and the filtrate was concentrated to dryness under reduced pressure to give 187 mg of N-[1-(3-(4-methoxyphenyl)propyl) 4 piperidyl]-3-(3,4-dihydroxyphenyl)-propionamide•hydrochloride (colorless powder, m.p. 89 - 93 °C). NMR Spectra ($CD_3OD$)
δ: 1.65 - 1.72 (2H, m), 1.95 - 2.13 (4H, m), 2.41 (2H, t, J = 7.4Hz), 2.64 (2H, t, J = 7.4Hz), 2.75 (2H, t, J = 7.4Hz), 2.95 - 3.15 (4H, m), 3.75 (3H, s), 3.80 - 3.94 (1H, m), 6.51 (1H, d, J = 7.8Hz), 6.61 - 6.78 (2H, m), 6.85 (2H, d, J = 8.5Hz), 7.14 (2H, d, J = 8.5Hz)
MASS Spectrum m/z 413 ($MH^+$)

Example 3

N-[1-(3-(3,4-dimethoxyphenyl)propyl)-4-piperidyl]-3,4-dihydroxycinnamide•hydrochloride, 178 mg, was dissolved in 5 ml of methanol, followed by degassing. To the solution was added 20 mg of 5% palladium charcoal and hydrogenation was performed at room temperature under normal pressure for 6 hours. After the reaction was completed, the palladium charcoal was filtered off and the filtrate solution was concentrated to dryness under reduced pressure. The residue was purified by preparative thin layer chromatography (PTLC) (chloroform : methanol = 80 : 20) to give 137 mg of N-[1-(3-(3,4-dimethoxyphenyl)propyl)-4-piperidyl] 3-(3,4-dihydroxyphenyl)propionamide•hydrochloride. NMR Spectrum ($CD_3OD$)
δ: 1.58 - 1.76 (2H, m), 1.88 - 2.05 (4H, m), 2.40 (2H, t, J = 7.3Hz), 2.63 (2H, t, J = 8.2Hz), 2.75 (2H, t, J = 7.3Hz), 2.80 - 3.01 (4H, m), 3.15 - 3.30 (1H, m), 3.79 (3H, s), 3.82 (3H, s), 6.47 - 6.70 (3H, m), 6.75 - 6.90 (3H, m)
MASS Spectrum m/z 442 (M+)

Example 4

To 6 ml of tetrahydrofuran were sequentially added 150 mg of 3,4-dihydroxybenzoic acid, 220 mg of 4-amino-1-(3-phenylpropyl)piperidine and 220 mg of dicyclo hexylcarbodiimide, and the mixture was subjected to heating and reflux for 4 hours. After cooling, precipitated crystalls were filtered off and the filtrate solution was concentrated under diminished pressure. The residue was purified by PTLC (chloroform : methanol = 90 : 10) to give 130 mg of N-[1-(3-phenylpropyl)-4-piperidyl-3,4-dihydroxybenzamide (colorless powder, m.p. 97 - 101 °C).
NMR Spectra ($CD_3OD$)
δ: 1.58 - 1.76 (2H, m), 1.78 - 2.01 (4H, m), 2.19 (2H, t, J = 11Hz,, 2.45 (2H, t, J = 7.7Hz), 2.63 (2H, t, J = 7.5Hz), 3.02 (2H, t, J = 11Hz), 3.79 - 3.93 (1H, m), 6.78 (1H, d, J = 8.3Hz), 7.09 - 7.31 (7H, m)
MASS Spectrum m/z 354 ($M^+$)

Example 5

3,4-Dihydroxyphenylacetic acid, 170 mg, was dissolved in 2 ml of dimethylformamide, and to the solution were added a solution of 220 mg of 4-amino-1-(3-phenylpropyl)piperidine in 1 ml of dimethylformamide and 140 mg of 1-hydroxybenzotriazole. After the resulting solution was cooled to - 10 °C, 210 mg of dicyclohexylcarbodiimide was added, and the mixture was stirred at - 10 °C for 3 hours and then at room temperature overnight. Precipitated crystalls were filtered off and the filtrate solution was concentrated under reduced pressure. The residue was purified by PTLC (chloroform : methanol = 90 : 10) to give 330

mg of N-[1-(3-phenylpropyl)-4-piperidyl]-3,4-dihydroxyphenylacetamide (m.p. 71 - 74° C).
NMR Spectra ($CD_3OD$)
δ: 1.40 - 1.64 (2H, m), 1.68 - 1.95 (4H, m), 2.11 (2H, t, J=10Hz), 2.39 (2H, t, J=7.4Hz), 2.49 (2H, t, J=7.4Hz), 2.92 (2H, d, J=11Hz), 3.55 - 3.73 (1H, m), 6.52 - 6.75 (3H, m), 7.05 - 7.31 (5H, m)
MASS Spectrum m/z 368 ($M^+$)

Example 6

N-[1-(3-Phenylpropyl)-4-piperidyl]-3-(3,4-dihydroxyphenyl)propionamide•hydrochloride, 100 mg, was dissolved in 570 mg of pyridine. Under ice cooling, 730 mg of anhydrous acetic acid was added dropwise and stirring was conducted at room temperature for 2 hours. The reaction solution was placed in ice water and extracted with chloroform, and the extract was washed with 1N hydrochloric acid, an aqueous saturated solution of sodium bicarbonate and an aqueous saturated solution of sodium chloride in sequence, followed by drying. The solvent was concentrated to dryness under diminished pressure to give 84 mg of N-[1-(3-phenylpropyl)-4-piperidyl]-3-(3,4-diacetoxyphenyl)propionamide.
NMR Spectra ($CD_3OD$)
δ: 1.30 - 1.49 (2H, m), 1.65 - 1.88 (4H, m), 2.01 (2H, t, J=11Hz), 2.23 (3H, s), 2.25 (3H, s), 2.35 (2H, t, J=8.0Hz), 2.44 (2H, t, J=7.4Hz), 2.60 (2H, t, J=7.6Hz), 2.70 - 2.93 (4H, m), 3.50 - 3.65 (1H, m), 6.98 - 7.28 (8H, m)
MASS Spectrum m/z 466 ($M^+$)

Further examples of the compounds of this invention are listed below, but should not be understood as limiting to them.

7) N-[1-(3-Phenylpropyl)-4-piperidyl]-4-(3,4-dihydroxyphenyl)butyramide (colorless powder, m.p. 181.5 - 182.5° C)
NMR Spectra ($CD_3OD$)
δ: 1.44 - 1.53 (2H, m), 1.77 - 1.87 (6H, m), 2.05 - 2.17 (4H, m), 2.36 - 2.48 (4H, m), 2.61 (2H, t, J=7.6Hz), 2.92 (2H, d, J=18Hz), 3.55 - 3.70 (1H, m), 6.48 (1H, d, J=8.0Hz), 6.59 (1H, d, J=2.0Hz), 6.65 (1H, d, J=8.0Hz), 7.11 - 7.27 (5H, m)
MASS Spectrum m/z 396 ($M^+$)

8) N-[1-(2-Phenylethyl)-4-piperidyl-3-(3,4-dihydroxyphenyl)propionamide
NMR Spectrum ($CD_3OD$)
δ: 1.55 - 1.90 (2H, m), 1.91 - 2.15 (2H, m), 2.42 (2H, t, J=6.9Hz), 2.77 (2H, t, J=7.4Hz), 3.00 - 3.27 (6H, m), 6.52 (1H, d, J=8.0Hz), 6.62 (1H, d, J=2.0Hz), 6.67 (1H, d, J=8.0Hz), 7.25 - 7.40 (5H, m)
MASS Spectrum m/z 368 ($M^+$)

9) N-(1-Benzyl-4-piperidyl)-3-(3,4-dihydroxyphenyl)propionamide
NMR Spectrum ($CD_3OD$)
δ: 1.30 - 1.52 (2H, m), 1.75 (2H, d, J=11Hz), 2.10 (2H, t, J=9.9Hz), 2.36 (2H, t, J=7.8Hz), 2.73 (2H, t, J=7.4Hz), 2.80 (2H, d, J=12Hz), 3.50 (2H, s), 3.53 - 3.60 (1H, m), 6.49 (1H, d, J=8.0Hz), 6.60 (1H, d, J=2.0Hz), 6.64 (1H, d, J=8.0Hz), 7.20 - 7.35 (5H, m)
MASS Spectrum m/z 354 ($M^+$)

10) N-[1-(4-Phenylbutyl)-4-piperidyl]-3-(3,4-dihydroxyphenyl)propionamide
NMR Spectrum ($CD_3OD$)
δ: 1.55 - 1.82 (6H, m), 1.90 - 2.10 (2H, m), 2.41 (2H, t, J=7.4Hz), 2.69 (2H, t, J=7.3Hz), 2.76 (2H, t, J=7.3Hz), 2.90 - 3.10 (4H, m), 3.77 - 3.90 (1H, m), 6.51 (1H, d, J=8.0Hz), 6.61 (1H, d, J=1.9Hz), 6.66 (1H, d, J=8.0Hz), 7.13 - 7.34 (5H, m)
MASS Spectrum m/z 396 ($M^+$)

11) N-[1-(3-(4-Chlorophenyl)propyl)-4-piperidyl]-3-(3,4-dihydroxyphenyl)propionamide
NMR Spectrum ($CD_3OD$)
δ: 1.55 = 1.80 (2H, m), 1.90 - 2.10 (4H, m), 2.41 (2H, t, J=7.1Hz), 2.69 (2H, t, J=7.5Hz), 2.75 (2H, t, J=7.3Hz), 2.95 - 3.15 (4H, m), 3.80 - 3.90 (1H, m), 6.51 (1H, d, J=8.0Hz), 6.61 (1H, s), 6.66 (1H, d, J=8.0Hz), 7.18 - 7.37 (4H, m)
MASS Spectrum m/z 416 ($M^+$)

12) N-[1-(3,3-Diphenyl)propyl-4-piperidyl]-3-(3,4-dihydroxyphenyl)propionamide
NMR Spectrum ($CD_3OD$)
δ: 1.55 - 1.76 (2H, m), 1.90 - 2.08 (2H, m), 2.39 (2H, t, J=7.2Hz), 2.43 - 2.57 (2H, m), 2.75 (2H, t, J=7.4Hz), 2.90 -3.12 (4H, m), 3.75 - 3.78 (1H, m), 4.01 (1H, t, J=8.0Hz), 6.50 (1H, d, J=8.0Hz), 6.60 (1H, d, J=2.0Hz), 7.15 - 7.38 (10H, m)

MASS Spectrum m/z 458 ($M^+$)

13) N-[1-(2-(3,4-Dimethoxyphenyl)ethyl)-4-piperidyl]-3-(3,4-dihydroxyphenyl)propionamide

NMR Spectrum ($CD_3OD$)

δ:1.38 - 1.57 (2H, m), 1.75 - 1.88 (2H, m), 2.18 (2H, t, J = 12Hz), 2.38 (2H, t, J = 7.6Hz), 2.52 -2.62 (2H, m), 2.68 -2.85 (4H, m), 2.92 (2H, d, J = 11.5Hz), 3.57 - 3.74 (1H, m), 3.79 (3H, s), 3.82 (3H, s), 6.50 (1H, d, J = 8.0Hz), 6.62 (1H, d, J = 2.0Hz), 6.66 (1H, d, J = 8.0Hz), 6.75 (1H, d, J = 8.1Hz), 6.81 (1H, d, J = 1.8Hz), 6.85 (1H, d, J = 8.2Hz)

MASS Spectrum m/z 429 ($MH^+$)

14) N-(4-Piperidyl)-3-(3,4-dihydroxyphenyl)propionamide

15) N-(1-Propyl-4-piperidyl)-3-(3,4-dihydroxyphenyl)propionamide

16) N-[1-(3-(3,4-Dihydroxy)phenylpropyl)-4-piperidyl]-3-(3,4-dihydroxyphenyl)propionamide

17) N-[1-(3-(3,4-dimethoxy)phenylpropyl)-4-piperidyl]-3-(3,4-diacetoxyphenyl)propionamide

18) N-[1-(3-Phenylpropyl)-4-piperidyl]-3-(3,4-di(N-n-butylcarbamoyloxy)phenyl)propionamide

NMR Spectrum ($CDCl_3$)

δ: 0.92 - 0.97 (6H, m), 1.28 - 1.47 (6H, m), 1.48 - 1.60 (4H, m), 1.65 - 1.90 (4H, m), 2.04 (2H, t, J = 12Hz), 2.32 - 2.43 (4H, m), 2.61 (2H, t, J = 7.7Hz), 2.81 (2H, d, J = 13Hz), 2.91 (2H, t, J = 7.3Hz), 3.21 - 3.28 (4H, m), 3.65 - 3.81 (1H, m), 5.05 - 5.15 (2H, m), 5.32 (1H, d, J = 7.9Hz), 6.98 - 7.13 (3H, m), 7.15 - 7.30 (5H, m)

MASS Spectrum m/z 580 ($M^+$)

19) N-(1-Benzyl-4-piperidyl)-5-(3,4-dihydroxyphenyl)valeramide

20) N-(4-Piperidyl)-4-(3,4-dihydroxyphenyl)butyramide

21) N-[1-(3-Phenylpropyl)-4-piperidyl]-(3,4-diacetoxyphenyl)butyramide

NMR Spectrum

δ: 1.53 - 1.70 (2H, m), 1.84 - 2.05 (6H, m), 2.19 (2H, t, J = 7.2Hz), 2.25 (3H, s), 2.26 (3H, s), 2.50 - 2.80 (8H, m), 3.21 (2H, d, J = 12Hz), 3.68 - 3.75 (1H, m), 7.00 - 7.46 (8H, m)

MASS Spectrum m/z 480 ($M^+$)

## Claims

1. A catechol derivative represented by the general formula:

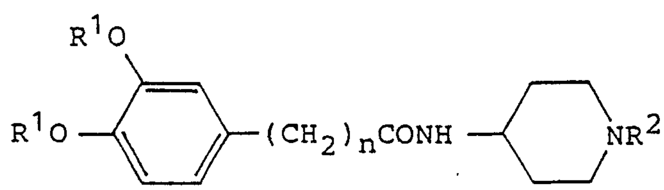

wherein $R^1$ is hydrogen atom, an acyl radical or a carbamoyl radical; $R^2$ is hydrogen atom, a lower alkyl radical or an aralkyl radical of the general formula:

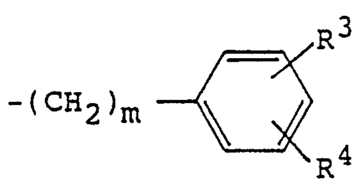

or

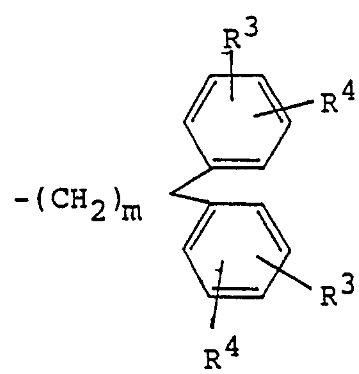

(wherein $R^3$ and $R^4$ may be the same or different substituents selected from the group consisting of hydrogen atom, a lower alkoxy, a halogen atom, a lower alkyl, trifluoromethyl and hydroxyl and m is an integer of 1 to 4), n is an integer of 1 to 4; and its pharmaceutically acceptable acid addition salt.

2. A derivative or a pharmaceutically acceptable salt thereof as claimed in claim 1 which is selected

from the group consisting of N-[1-(3-phenylpropyl) 4-piperidyl]-3-(3,4-dihydroxyphenyl)-propionamide•hydrochloride, N-[1-(3-(4- methoxyphenyl)propyl)-4-piperidyl-3-(3,4-dihydroxyphenyl)-propionamide•hydrochloride, N-[1-(3-(3,4-dimethoxyphenyl)-4-piperidyl]-3-(3,4-dihydroxyphenyl)-propionamide•hydrochloride, N-[1-(3-phenylpropyl)-4-piperidyl]3,4-dihydroxyphenylacetamide, N-[1-(3-phenylpropyl)-4-piperidyl]-3-(3,4-diacetoxyphenyl)propionamide, N-[1-(3-phenylpropyl)-4-piperidyl]-4-(3,4-dihydroxyphenyl)butyramide, N-[1-(2-phenylethyl)-4-piperidyl]-3-(3,4-dihydroxyphenyl)propionamide, N-[1-(4-phenylbutyl)-4-piperidyl]-3-(3,4-dihydroxyphenyl)propionamide, N-[1-(3-(4-chlorophenyl)propyl)-4-piperidyl]-3-(3,4-dihydroxyphenyl)propionamide, N-[1-(3,3-diphenyl)propyl-4-piperidyl]-3-(3,4-dihydroxyphenyl)propionamide, N-[1-(2-(3,4-dimethoxyphenyl)ethyl)-4-piperidyl]-3-(3,4-dihydroxyphenyl)-propionamide, N-[1-(3-phenylpropyl)-4-piperidyl-(3,4-diacetoxyphenyl)butyramide.

3. A pharmaceutical composition for the prevention or treatment of degenerative neurological disorders comprising a therapeutically effective amount of a derivative of claim 1 or Claim 2 and pharmaceutically acceptable additives.

Claims for the following Contracting State: ES:

1. A method of producing a catechol derivative represented by the general formula:

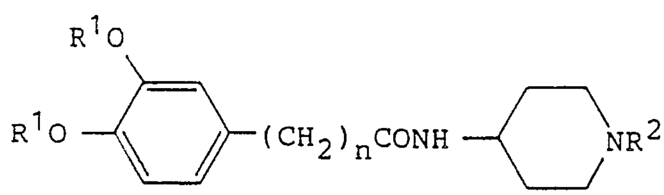

wherein n is an integer of 1 to 4; $R^1$ is hydrogen atom, an acyl radical or a carbamoyl radical; $R^2$ is hydrogen atom, a lower alkyl radical or an aralkyl radical or the formula:

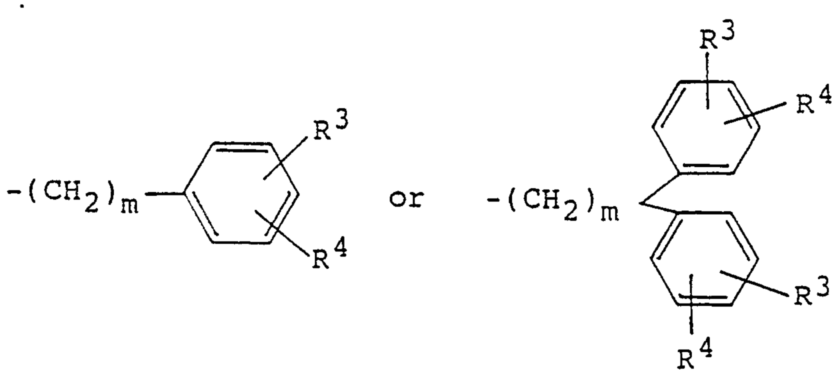

(wherein $R^3$ and $R^4$ may be the same or different substituents selected from the group consisting of hydrogen atom, a lower alkoxy, a halogen atom, a lower alkyl, trifluoromethyl and hydroxyl and m is an integer of 1 to 4) or its acid addition salt, which comprises hydrogenating a compound of the formula:

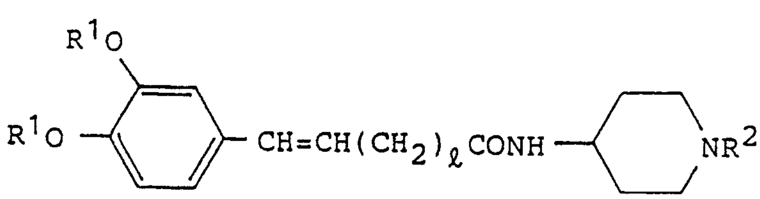

wherein $\ell$ is an integer of 0 to 2, and $R^1$ and $R^2$ are as described above.

2. A method of producing a catechol derivative as claimed in Claim 1 which comprises making to react a compound of the formula:

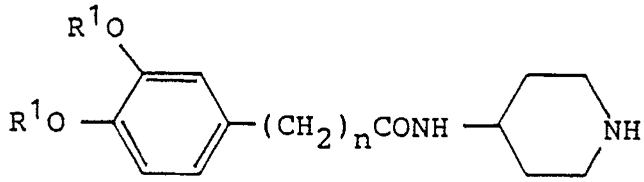

and a compound of the formula : $R^2$-X
wherein R' and n are the same as in the definition of Claim 1; $R^2$ is the same as in the definition of Claim 1 except for hydrogen atom; and X is a halogen atom, active esters, etc.

3. A method of producing a catechol derivative as claimed in Claim 1 wherein $R^1$ an acyl radical, which comprises making to react a compound of the formula:

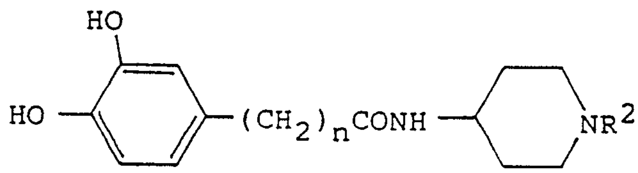

wherein $R^2$ and n are the same as in the definition of Claim 1, and a corresponding lower aliphatic acid or its acid anhydride from which the acyl radical is derived.

4. A method of producing a catechol derivative as claimed in Claim 1 wherein $R^1$ is a carbamoyl radiacal, which comprises making to react a compound of the formula:

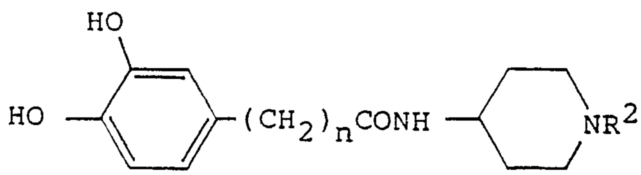

wherein $R^2$ and n are the same as in the definition of Claim 1, and a lower alkyl-substituted isocyanate.